(19) **Europäisches Patentamt**
European Patent Office
Office européen des brevets

(11) **EP 2 231 046 B1**

(12) **EUROPÄISCHE PATENTSCHRIFT**

(45) Veröffentlichungstag und Bekanntmachung des Hinweises auf die Patenterteilung:
**09.03.2016 Patentblatt 2016/10**

(51) Int Cl.:
***A61B 18/00*** *(2006.01)*

(21) Anmeldenummer: **08864067.7**

(22) Anmeldetag: **17.12.2008**

(86) Internationale Anmeldenummer:
**PCT/EP2008/010785**

(87) Internationale Veröffentlichungsnummer:
**WO 2009/080273 (02.07.2009 Gazette 2009/27)**

(54) **PLASMA-APPLIKATOREN FÜR PLASMACHIRURGISCHE VERFAHREN**

PLASMA APPLICATORS FOR PLASMA-SURGICAL METHODS

APPLICATEURS DE PLASMA POUR PROCÉDÉ DE CHIRURGIE AU PLASMA

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.12.2007 DE 102007061482**
**17.01.2008 DE 102008004843**

(43) Veröffentlichungstag der Anmeldung:
**29.09.2010 Patentblatt 2010/39**

(73) Patentinhaber: **Farin, Günter**
**72070 Tübingen (DE)**

(72) Erfinder: **Farin, Günter**
**72070 Tübingen (DE)**

(74) Vertreter: **Winter, Brandl, Fürniss, Hübner, Röss, Kaiser, Polte - Partnerschaft mbB Patent- und Rechtsanwaltskanzlei Bavariaring 10 80336 München (DE)**

(56) Entgegenhaltungen:
**WO-A-2007/047122        US-A1- 2004 116 918**
**US-A1- 2007 239 156        US-B1- 6 958 063**

EP 2 231 046 B1

**Beschreibung**

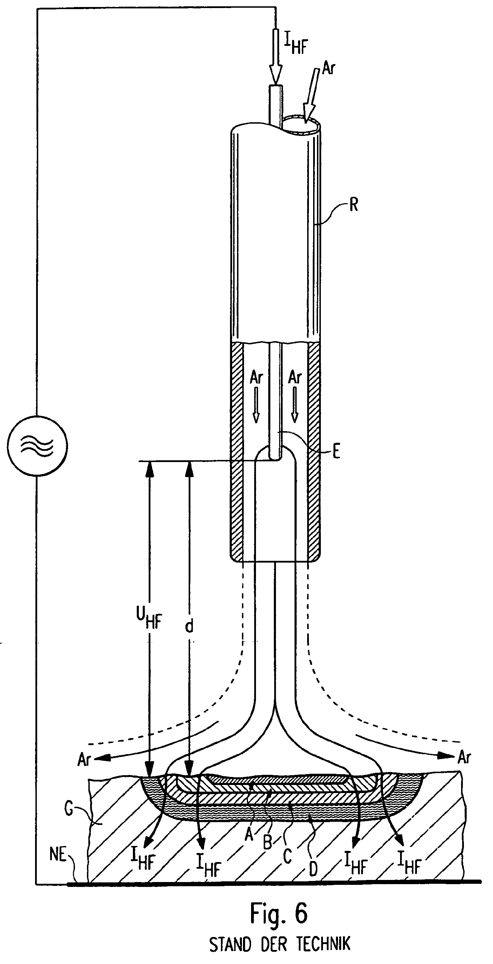

Die Erfindung betrifft Plasma-Applikatoren zur Anwendung der Plasmachirurgie in der Endoskopie sowie Plasma-Sonden zur Anwendung plasmachirurgischer Verfahren in der flexiblen Endoskopie. Mit Plasmachirurgie sind hier hochfrequenzchirurgischen Verfahren gemeint, bei welchen, wie in Figur 6 schematisch dargestellt, hochfrequenter elektrischer Wechselstrom (HF-Strom, $I_{HF}$) durch ionisiertes und folglich elektrisch leitfähiges Gas (Plasma), beispielsweise Argon-Plasma, gezielt auf oder in zu behandelndes biologisches Gewebe (Zielgewebe, A, B, C, D) appliziert wird um auf oder im Zielgewebe medizinisch nützliche thermische Effekte, insbesondere den Devitalisationseffekt (D), Koagulationseffekt (C), Desikkationseffekt (B) und/oder Schrumpfungseffekt (A) zu erzeugen, ohne jedoch kollaterales Gewebe (Kollateralgewebe, G) mehr als unvermeidlich und tolerabel zu schädigen. Die Ionisation des Gases zwischen einer Ionisationselektrode (E) und dem Zielgewebe entsteht bei ausreichend hoher elektrischer Feldstärke ($F_e$) entsprechend der Funktion $F_e = U_{HF} : d$. Zur Ionisation von Argon bei atmosphärischem Druck sind ca. 500 V/mm erforderlich. Der konstruktive Aufbau verschiedener Plasma-Applikatoren bzw. Plasma-Sonden (R) wird weiter unten ausführlich beschrieben.

Plasmachirurgische Verfahren und Plasma-Applikatoren sowie Einrichtungen zu deren Anwendung sind nicht neu. Bereits seit mehr als 50 Jahren wird ein unter der Bezeichnung Fulguration oder Spray-Koagulation bekanntes plasmachirurgisches Verfahren insbesondere zur thermischen Hämostase bei chirurgischen Operationen angewendet. Hierbei wird das Plasma ausschließlich in Luft erzeugt, d.h. es entstehen vorwiegend Sauerstoffplasma und Stickstoffplasma. Beide Plasmen sind bekanntlich chemisch sehr reaktiv und erzeugen an der Gewebeoberfläche Karbonisationseffekte, Pyrolyseeffekte und folglich auch Vaporisation von Gewebe und Rauch. Diese unbeabsichtigten Nebeneffekte der Fulguration bzw. Spray-Koagulation stören bzw. behindern die Anwendung dieses plasmachirurgischen Verfahrens insbesondere bei endoskopischen Operationen.

Die US 4,060,088 hat u.a. die Vermeidung der oben aufgeführten Nebeneffekte der Fulguration bzw. Spray-Koagulation zum Gegenstand. Dort wird vorgeschlagen, die Luft zwischen der Aktivelektrode und dem zu behandelnden Gewebe durch ein chemisch inertes Gas bzw. Edelgas zu ersetzen. Als Edelgas werden Helium oder Argon sowie Gemische hiervon vorgeschlagen. Wegen seines relativ geringen Preises wird heute jedoch vorwiegend Argon angewendet und dieses Verfahren zwecks Abgrenzung zu der in Luft stattfindenden Fulguration bzw. Spray-Koagulation seit ca. 20 Jahren Argon-Plasma-Koagulation (APC) genannt. Eine klinisch anwendbare Einrichtung hierfür wurde in der US 4,781,175 und zwar ausschließlich zur Anwendung in der offenen Chirurgie und in der starren Endoskopie zur thermischen Blutstillung vorgeschlagen.

Für die APC geeignete Einrichtungen und Verfahren sind erstmals 1994 von G. Farin und K.E. Grund beschrieben worden (G. Farin, K.E. Grund: Technology of Argon Plasma Coagulation with Particular Regard to Endoscopic Applications. Endoscopic Surgery and Allied Technologies, No. 1 Volum 2, 1994: 71-77; sowie K.E. Grund, D. Storek, G. Farin: Endoscopic Argon Plasma Coagulation (APC): First Clinical Experiences in Flexible Endoscopy. Endoscopic Surgery and Allied Technologies, No. 1 Volum 2, 1994: 42-46). Das Anwendungsspektrum der APC in der flexiblen Endoskopie wurde früher beschrieben (K.E. Grund, C. Zindel, G. Farin: Argonplasmakoagulation in der flexiblen Endoskopie: Bewertung eines neuen therapeutischen Verfahrens nach 1606 Anwendungen. Deutsche Medizinische Wochenschrift 122, 1977: 432-438) und gehört heute weltweit zum Standard nicht nur in der flexiblen Endoskopie.

Wie in oben zitierten Publikationen beschrieben, wurde die Argon-Plasma-Koagulation nicht nur zur Koagulation biologischer Gewebe angewendet. Heute wird dieses Verfahren insbesondere zur thermische Devitalisierung pathologische Gewebe und/oder zur Desikkation und folglich Schrumpfung von Blutgefäßen und deren kollateralem Gewebe zum Zwecke der Hämostase angewendet. Immer wichtiger wird die Anwendung dieses Verfahrens zur thermischen Devitalisierung relativ dünner Gewebeschichten, wie beispielsweise der Mukosa das Gastrointestinaltrakts oder Tracheobronchialsystems. Immer wichtiger wird dieses Verfahren auch zur thermischen Sterilisierung von Gewebeoberflächen bei transmuralen Operationen, beispielweise bei transgastralen Operationen, um Keimdisseminationen aus dem Magen in die Bauchhöhle zu vermeiden. Diese Anwendungen werden von der Bezeichnung Argon-Plasma-Koagulation (APC) nicht adäquat erfasst. Da der Gegenstand dieser Erfindung nicht nur auf die Koagulation biologischer Gewebe und auch nicht nur auf das Gas Argon begrenzt ist, wird dieses Verfahren im Folgenden entsprechend umfassender "Plasmachirurgie" genannt.

Das breite Anwendungsspektrum der Plasmachirurgie stellt heute allerdings differenziertere anwendungsspezifische Anforderungen an die Eigenschaften der hierfür erforderlichen Einrichtungen, insbesondere bezüglich Reproduzierbarkeit der beabsichtigten thermischen Effekte auf bzw. in Zielgeweben sowie der Vermeidung unbeabsichtigter thermischer Effekte sowohl im Zielgewebe als auch in kollateralen Geweben als dies früher bei Anwendungen der Fulguration bzw. Spray-Koagulation der Fall war. Dies gilt insbesondere bei Anwendung der Plasmachirurgie an bzw. in dünnwandigen Hohlorganen des Gastrointestinaltrakts oder des Tracheobronchialsystems (siehe hierzu G. Farin, K.E. Grund: Principles of Electrosurgery, Laser, and Argon Plasma Coagulation with Particular Regard to Colonoscopy. In: Colonoscopy; Principles and Practice, Edited by J.D. Waye, D.K. Rex and C.B. Williams, Blackwell Publishing 2003: 393-409.

Das Spektrum verschiedener Plasma-Applikatoren für medizinische Anwendungen, insbesondere für die Plasmachirurgie sowie für endoskopisch kontrollierte Interventionen, ist sehr breit. Bezüglich des Zugangs zu dem jeweils zu behandelnden Zielorgan bzw. Zielgewebe kann man die bisher verfügbaren Plasma-Applikatoren in solche differenzieren, die für die offene Chirurgie, für die starre Endoskopie und für die flexible Endoskopie gestaltet sind. Der prinzipielle Aufbau sowie die Funktion dieser verschiedenen Plasma-Applikatoren ist beispielsweise aus der Publikation von G. Farin und K.E. Grund: Technology of Argon Plasma Coagulation with Particular Regard to Endoscopic Applications, Endoscopic Surgery and Allied Technologies, Thieme Verlag, Stuttgart, No. 1, Volume 2, 1994: 71 - 77, bekannt.

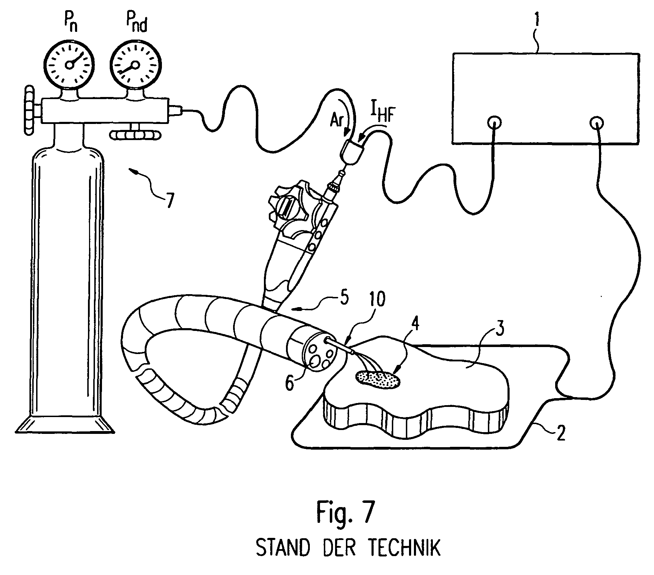

Eine Anordnung für die Plasmachirurgie, und zwar für endoskopisch kontrollierte Interventionen ist in Fig. 7 dargestellt. Eine solche Anordnung umfasst üblicherweise einen chirurgischen Hochfrequenzgenerator 1, der einerseits an eine Neutralelektrode 2 und andererseits an ein chirurgisches Instrument, in diesem Fall eine Sonde 10 (bzw. deren nicht gezeigte Entladungselektrode) angeschlossen ist. Die Sonde 10 steckt in einem der Arbeitskanäle 6 eines Endoskops 5. Einem Lumen der Sonde 10 wird Argon (oder ein anderes Edelgas) aus einer Edelgasquelle 7 zugeführt. Die Neutralelektrode 2 wird großflächig an einem Patienten platziert und steht so mit dessen biologischem Gewebe 3 in Verbindung. Auf diese Weise kann der Operateur ein Zielgewebe 4 mittels eines Argon-Plasmas behandeln, um die gewünschten Effekte, die anhand von Fig. 6 bereits beschrieben wurden, dort zu erzielen.

Die für die Plasmachirurgie verfügbaren HF-Generatoren kann man bezüglich ihres Innenwiderstands differenzieren. HF-Generatoren mit hohem Innenwiderstand eignen sich insbesondere für die Behandlung oberfläche Läsionen, bei welchen eine geringe Penetrationstiefe der thermischen Effekte zweckmäßig ist. HF-Generatoren mit kleinem Innenwiderstand eignen sich insbesondere für die Behandlung massiver Läsionen, bei welchen eine große Penetrationstiefe der thermischen Effekte zweckmäßig ist. In DE 198 39 826 ist ein HF-Generator beschrieben, dessen Innenwiderstand zwischen hoch und gering einstellbar ist.

Die Behandlung oberflächlicher Läsionen, bei welchen eine geringe Penetrationstiefe der thermischen Effekte zweckmäßig oder gar Voraussetzung sind, ist mit HF-Generatoren mit geringem Innenwiderstand und bekannten Plasma-Applikatoren insofern problematisch, als die Penetrationstiefe der thermischen Effekte vorwiegend durch die Applikationsdauer, also vom Operateur, kontrolliert werden muss. Da die Penetrationsgeschwindigkeit der thermischen Effekte während des Anfangs der Plasma-Applikation relativ schnell ist und über der Zeit degressiv bis zum Erreichen der maximal erreichbaren Penetrationstiefe langsamer wird, ist eine geringe und gleichmäßige Penetrationstiefe der thermischen Effekte bei flächigen Läsionen nur schwierig oder gar nicht realisierbar. Die Penetrationsgeschwindigkeit der thermischen Effekte kann zwar durch Variation der Leistung bzw. des durch das Plasma fließenden HF-Stroms beeinflusst werden, dies wird mit Rücksicht auf die zum Ionisieren des Gases erforderlichen hohen HF-Spannung bei bekannten HF-Generatoren durch Impulsmodulation der HF-Spannung bzw. des HF-Stroms realisiert. Die Pulsmodulation kann jedoch Videosignale von Video-Endoskopen stören und neuromuskuläre Reize verursachen, letzteres insbesondere bei Modulationsfrequenzen unterhalb 1 kHz. Ein weiteres Problem bei der Anwendung von HF-Generatoren mit geringem Innenwiderstand zur Behandlung oberflächlicher Läsionen sind sehr hohe Temperatur des Plasmas infolge der zum Ionisieren erforderlichen hohen HF-Spannung einerseits und des geringen Innenwiderstand des HF-Generators sowie des geringen elektrischen Widerstands der Plasmastrecke andererseits, wodurch in der Plasmastrecke eine sehr hohe HF-Stromdichte und folglich derart hohe Temperatur verursacht werden können, dass die bei diesen Anwendung störenden Karbonisations- und Pyrolyseeffekte entstehen können.

HF-Generatoren mit hohem Innenwiderstand sind für endoskopisch Operationen bzw. Interventionen nicht zweckmäßig oder gar nicht anwendbar, weil die für die Ionisation des Gases zwischen Ionisationselektrode und Zielgewebe erforderliche hohe HF-Spannung infolge der sogenannten Streukapazität zwischen aktiver HF-Leitung und neutraler HF-Leitung nur ungenügend oder gar nicht vom HF-Generator auf die Ionisationselektrode übertragen werden kann. Dies ist bekanntlich insbesondere bei der flexiblen Endoskopie der Fall.

HF-Generatoren entsprechend DE 198 39 826 sind bisher nicht verfügbar.

Die EP 1 148 770 A2 und die US 6 958 063 B1 beschreiben einen Plasma-Applikator für plasmachirurgische Verfahren, der insofern auf einem anderen Prinzip als der erfindungsgemäße Plasmaapplikator arbeitet, als keine in der üblichen Weise vorgesehene Neutralelektrode verwendet wird. Der dort vorgesehene HF-Generator soll ein Resonanzübertrager sein, wobei der HF-Strom als "dielektrischer Verschiebungsstrom von der Oberfläche des Patienten nach Masse" abfließt. Dadurch soll eine Verschorfung mit einem chirurgischen Kaltplasmastrahlgerät erzeugt werden, die soweit wie möglich ohne Verkohlung oder Verbrennungsprodukte aufgrund von Sauerstoffeinschlüssen abläuft.

Zur Lösung dieser Aufgabe wird in der Druckschrift vorgeschlagen, im Handstück des Gerätes einen Kondensator in Reihenschaltung zum Stromkreis vorzusehen. Dieser Kondensator ist in seinem Anbringungsort festgelegt. Auch dann, wenn zum Beispiel eine Leitung zwischen dem Handstück und der Elektrode vorgesehen ist, verbleibt der Kondensator gemäß dem Vorschlag dieser Druckschrift im Handstück.

Ausgehend vom oben genannten Stand der Technik liegt der Erfindung die Aufgabe zu Grunde, ein elektrochirurgisches Instrument der eingangs genannten Art dahin gehend aufzuzeigen, dass mit geringem Aufwand und dennoch mit großer Sicherheit eine hinreichend geringe Penetrationstiefe erzielbar ist.

Diese Aufgabe wird durch ein Plasmaapplikator nach Patentanspruch 1 gelöst. Der Plasmaapplikator umfasst eine rdw - oder schlauchförmig ausgebildete Sonde, die zum Einführen in einen Arbeitskanal eines flexiblen Endoskops vorgesehen ist.

Insbesondere wird die Aufgabe durch ein Plasmaapplikator zum Übertragen elektrischer Energie von einem elektrochirurgischen HF-Generator über eine Anschlussleitung und eine an deren distalem Ende angeschlossene Elektrode und weiter über einen Strompfad aus ionisiertem Gas in ein biologisches Zielgewebe erreicht, wobei zwischen dem distalen Ende der Anschlussleitung und der Elektrode ein Widerstandselement mit vorbestimmter Impedanz angeordnet ist, die derart dimensioniert ist, dass nach der Ionisierung des Gases eine Begrenzung eines Behandlungsstromes sichergestellt ist.

Ein wesentlicher Punkt der Erfindung liegt also darin, dass die gesamte Anordnung, bestehend aus HF-Generator und allen Zuleitungen bis hin zur Elektrode als "Gesamtgenerator" betrachtet wird, dessen Innenwiderstand dann durch das Widerstandselement bestimmt ist. Dieses Widerstandselement kann nun entsprechend den Anforderungen an die zu erzielende Penetrationstiefe gewählt werden bzw. man kann verschiedene Instrumente für verschiedene Penetrationstiefen zur Verfügung stellen. Selbstverständlich spielt nach wie vor die Behandlungszeit eine Rolle, jedoch ist gerade die kritische Phase, nämlich der kurze Moment nach dem "Zünden" des Lichtbogens, während derer ein hoher Strom fließen kann, entschärft.

Das Widerstandselement kann ein ohmscher Widerstand sein, der die angestrebte Strombegrenzung sicherstellt. Vorzugsweise wird jedoch das Widerstandselement als Kapazität ausgebildet bzw. umfasst eine solche Kapazität, welche die hier notwendige Spannungsfestigkeit aufweist. Der große Vorteil liegt in diesem Fall darin, dass diese Kapazität einen Hochpass bildet, so dass niederfrequente Anteile des Stromes gedämpft werden. Dies wiederum führt zu einer erheblichen Verminderung von Störungen von Videosystemen (wie sie in Endoskopen heute üblicherweise verwendet werden) und zur Vermeidung von neuromuskulären Reizungen, wie sie durch niedrigere Frequenzen auftreten können.

Bei ausreichend großen Plasma-Applikatoren kann der kapazitive Widerstand durch handelsübliche Bauelemente, beispielsweise hochspannungsfeste Keramikkondensatoren, realisiert werden. Bei Plasma-Applikatoren der Erfindung, die durch enge Instrumentierkanäle flexibler Endoskope (wie in Zusammenhang mit Fig. 7 beschrieben) angewendet werden, wie beispielsweise Plasma-Applikatoren bzw. sogenannte Argon-Plasma-Coagulations-Sonden (APC-Sonden), z.B. entsprechend DE 198 20 240 oder DE 101 29 699 bzw. EP 1 397 082, die einen Außendurchmesser von nur 2 bis 3,5 mm haben, müssen diese kapazitiven Widerstände jedoch speziell für diese Plasma-Applikatoren entwickelt oder konstruktiv durch geeignete Gestaltung des distalen Endes der APC-Sonden realisiert werden.

Da diese bisher sogenannten APC-Sonden selbstverständlich nicht nur mit Argon und auch nicht nur zur Koagulation, sondern auch mit anderen Gasen oder Gasgemischen, für andere thermische Effekte und gegebenen Falls auch in anderen Fachbereichen angewendet werden können, werden diese Plasma-Applikatoren im Folgenden allgemeingültiger Plasma-Sonden (P-Sonden) genannt.

Bei einer bevorzugten Ausführungsform umfasst das Widerstandselement einen Teilabschnitt der in der sonde angeordneten Anschlusleitung und/oder der Elektrode. Es werden also Abschnitte dieser Bauteile entweder zur Bildung eines ohmscheil Widerstands oder (gegebenenfalls zusätzlich) zur Bildung einer Kapazität verwendet. Dies kann zum Beispiel dadurch geschehen, dass das Widerstandselement durch elektrisch gegeneinander isolierte, parallel geführte oder verdrillte oder koaxial angeordnete Abschnitte der Anschlussleitung und/oder einer Zuleitung zur Elektrode und/oder der Elektrode selbst gebildet wird. Ein solcher Aufbau ist relativ einfach. In diesem Fall sollte auch darauf geachtet werden, dass keine Induktivitäten entstehen, was beispielsweise durch Verwendung biphilarer Leitungsanordnungen geschehen kann.

Vorzugsweise weist das Widerstandselement eine Kapazität von 10 pF bis etwa 1.000 pF auf. Dies sind Kapazitätsbereiche, die bei den in der Hochfrequenzchirurgie üblicherweise verwendeten Frequenzen zu Strömen führen, welche die gewünschte relativ niedrige Penetrationstiefe sicherstellen.

Das für die Bildung der Kapazität verwendete Dielektrikum sollte eine möglichst hohe Dielektrizitätskonstante aufweisen. Hierfür eignen sich nicht nur Kunststoffe, sondern vor allem auch Keramikmaterial, das als Isolierung und/oder Dielektrikum eingebracht wird. Dies kann starres Keramikmaterial oder aber (wenn eine höhere Flexibilität gefordert ist) auch pulverförmiges Keramikmaterial sein.

Alle obigen Punkte gelten auch für solche elektrochirurgischen Anordnungen, in denen kein "Schutzgas" verwendet wird. Vorzugsweise wird jedoch mit einem Schutzgas, einem Edelgas (insbesondere Argon) gearbeitet. Die Elektrode befindet sich der Sonde und ist derart positioniert, dass ein Edelgas, insbesondere Argon, als zu ionisierendes Gas in einen Raum zwischen der Elektrode und dem Zielgewebe zuführbar ist. Die hieraus resultierenden Vorteile wurden oben bereits beschrieben.

Nachfolgend wird die Erfindung anhand von Abbildungen näher erläutert. Hierbei zeigen

- Fig. 1 eine schematisierte Darstellung einer Ausführungsform des elektrochirurgischen Instruments,

- Fig. 2-5 schematisierte Darstellungen als kapazitive Elemente ausgebildeten Widerstandselementen und den dazu gehörigen Elektroden,

- Fig. 6 die eingangs angesprochene Darstellung zur Erläuterung der Vorgänge bei der Argon-Plasma-Koagulation und

- Fig. 7 eine Gesamtanordnung zur endoskopischen Behandlung von Geweben mittels APC.

In der nachfolgenden Beschreibung werden für gleiche und gleich wirkende Gegenstände dieselben Bezugsziffern verwendet.

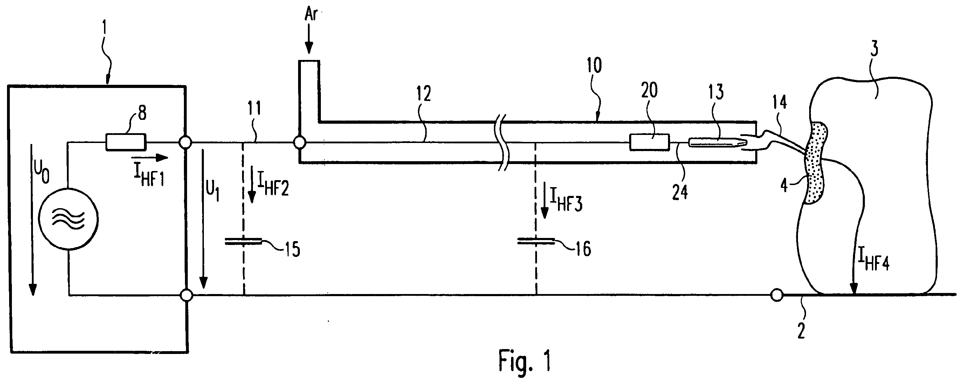

In Fig. 1 ist stark schematisiert eine Anordnung gezeigt, die im Prinzip derjenigen aus Fig. 7 entspricht. Das in Fig. 7 gezeigte Endoskop ist in dieser Anordnung nicht zu sehen.

Wie in Fig. 1 gezeigt, ist ein Hochfrequenzgenerator vorgesehen, der eine Spannungsquelle mit einer Spannung $U_0$ und einen Innenwiderstand 8 mit einem Widerstandswert R; aufweist. Somit steht bei einem Ausgangsstrom $I_{HF1}$ am Ausgang des Generators 1 eine Spannung $U_1$

$$U_1 = U_0 - R_i * I_{HF1}$$

an den Ausgangsklemmen des Generators 1 an. Der Generator ist über eine Zuleitung 11 mit einer Sondenzuleitung 12 verbunden, die innerhalb eines Schlauches der Sonde 10 angeordnet ist. Die Sondenleitung 12 ist an ihrem distalen Ende über ein Widerstandselement 20 und eine Elektrodenzuleitung 24 mit einer Elektrode 13 verbunden. Durch den Schlauch der Sonde 10 wird Argongas Ar geleitet, so dass ein Raum zwischen dem distalen Ende der Sonde 10 und dem biologischen Gewebe 3 mit Argongas gefüllt, normalerweise dort befindliche Luft also verdrängt wird. Wenn die Spannung zwischen der Spitze der Elektrode 13 und dem biologischen Gewebe 3 hoch genug ist, wird das Gas (Argon) in diesem Raum zwischen der Elektrode 13 und dem biologischen Gewebe 3 ionisiert, so dass ein Lichtbogen 14 entsteht. Dann fließt ein Strom $I_{HF4}$ durch das Zielgewebe 4 und das biologische Gewebe 3 zur Neutralelektrode 2.

Die Zuleitung 11 wird üblicherweise als monopolare Leitung ausgebildet. Weiterhin liegt die Neutralelektrode 2 auf Umgebungspotential (ebenso wie ein gegebenenfalls vorgesehenes Endoskop), so dass sich eine relativ große Streukapazität 15 zwischen der Zuleitung 11 sowie eine Streukapazität 16 zwischen der Sondenleitung 12 und der Umgebung bildet. Durch diese Streukapazitäten 15 und 16 fließen Ströme $I_{HF2}$ bzw. $I_{HF3}$. Durch diese Streukapazität sinkt schon vor Zünden eines Lichtbogens 14 die Spannung ($U_z$) ab, die zwischen der Elektrode 13 und dem Zielgewebe 4 zum Zünden Plasmas gebraucht wird:

$$U_Z = U_0 - R_i (I_{HF2} + I_{HF3})$$

Um nun die Zündung des Plasmas 14 bei einem möglichst großen Abstand zwischen der Elektrode 13 und dem Zielgewebe 4 sicherzustellen, ist es von Vorteil, wenn der Betrag $R_i$ des Innenwiderstandes 8 gering ist. Andererseits wird in diesem Fall bei einem Zünden des Lichtbogens 14, der einen sehr geringen Widerstand hat, sowie aufgrund der Tatsache, dass der Widerstand zwischen dem Zielgewebe 4 und der Neutralelektrode 2 ebenfalls relativ gering ist, ein sehr großer Strom $I_{HF4}$ fließen, so dass in kurzer Zeit das Zielgewebe 4 bis in eine relativ große Tiefe beeinflusst wird. Dadurch nun, dass der Widerstand 20 zwischen dem distalen Ende der (verlustbehafteten) Leitung 11, 12 und der Elektrode 13 angeordnet ist, wird selbst bei einer hohen, an der Elektrode 13 zur Verfügung stehenden Zündspannung, nach dem Zünden des Lichtbogens 14 ein hoher Spannungsabfall erzeugt, so dass der Strom $I_{HF4}$ begrenzt werden kann. Eine solche Begrenzung des Stromes ist nur durch ein Widerstandselement 20 an dieser Stelle möglich. Es soll hierbei aber unterstrichen werden, dass das Widerstandselement 20 keineswegs ein lokal begrenztes Widerstandselement sein muss. Dieses Widerstandselement 20 kann in verschiedener Weise sich über eine gewisse Länge bis zur Spitze der Elektrode 13 erstrecken.

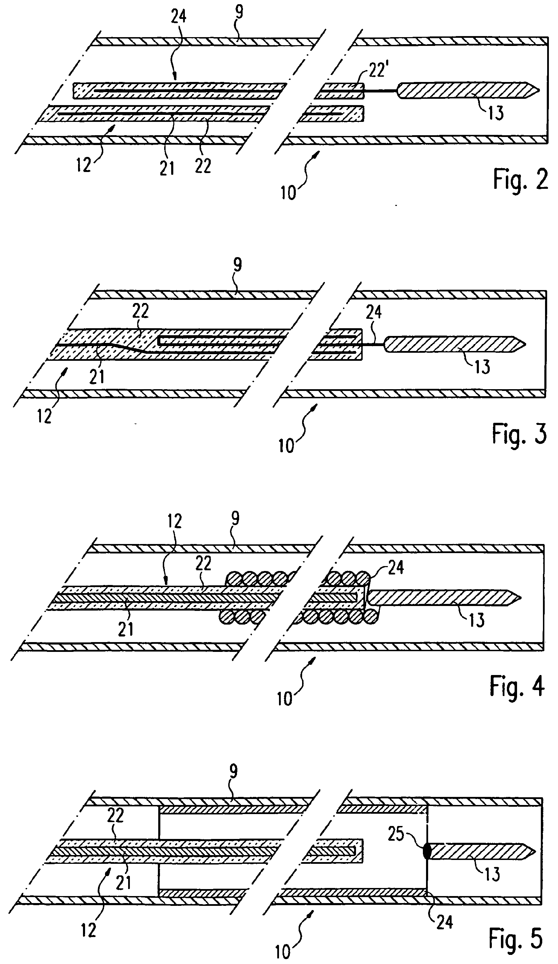

Nachfolgend werden verschiedene Ausführungsformen des Widerstandselements anhand der Fig. 2-5 erläutert.

Bei der in Fig. 2 gezeigten Ausführungsform ist ein distales Ende der Sondenleitung 12 gezeigt, die einen Sondenleitungsdraht 21 aufweist, der über ein Isoliermaterial 22 isoliert ist. Parallel zu diesem distalen Ende der Sondenleitung 12 ist die Elektrodenzuleitung 24 angeordnet, die mit der Elektrode 13 verbunden und mit einer Isolierung 22' versehen ist. Durch diese Parallelführung der beiden Leitungen 12/24 wird eine Kapazität gebildet, die als Widerstandselement 20 fungiert.

Die in Fig. 3 gezeigte Ausführungsform unterscheidet sich von der nach Fig. 2 dadurch, dass sowohl das distale Ende des Sondenleitungsdrahts 21 als auch das Ende der Elektrodenzuleitung 24 in einem gemeinsamen Isolationsmaterial 22 eingebettet sind.

Darüber hinaus ist die Elektrodenzuleitung 24 biphilar ausgebildet, so dass gegebenenfalls entstehende Leitungsinduktivitäten kompensiert werden. Als Isolationsmaterial eignet sich gerade in einem solchen Fall ein Keramikmaterial (als massive oder als pulverförmige Keramik ausgebildet), um eine möglichst hohe Kapazität bei kleiner Bauform der Anordnung zu erzielen.

Bei der in Fig. 4 gezeigten Ausführungsform wird die Kapazität dadurch erhöht, dass die Elektrodenzuleitung 24 um das Ende der Sondenzuleitung 12 gewickelt ist. Auch hier kann wieder eine biphilare Anordnung zur Vermeidung von Induktivitäten gewählt werden.

Bei der in Fig. 5 gezeigten Anordnung ist die Elektrodenzuleitung 24 als Hülse ausgebildet, welche das distale Ende der Sondenleitung 12 umgibt und so mit dieser eine Kapazität bildet. In diesem Fall ist die Elektrode über eine Verbindungsstelle 25 mit der hülsenförmigen Elektrodenzuleitung 24 elektrisch leitend verbunden. Die Dimensionierung kann auch ähnlich der nach Fig. 4 erfolgen, so dass zugeleitetes Argongas nicht mehr durch die hülsenförmige Elektrodenzuleitung 24, sondern außen an dieser vorbei in den Schlauch 9 der Sonde 10 strömt.

Wichtig bei allen Anordnungen ist eine entsprechende Isolation, so dass kein Durchschlag zwischen den durch Leitungen gebildeten Elementen erfolgt. Weiterhin ist es auch möglich, die Leitungen in eine Wand des Schlauches 9 einzubetten, der die Gasleitung der Sonde 10 bildet. Hierbei sei auch angemerkt, dass auch Arbeitskanal 6 des Endoskopes 5 als Gasleitung verwendet werden kann, wie dies beispielsweise in der EP 0 954 246 A1 beschrieben ist.

Die physikalischen Parameter, welche die Kapazität zwischen den Leitungen bestimmen, sind in der einschlägigen Fachliteratur detailliert beschrieben und sind dem Durchschnittsfachmann bekannt.

Die Anordnung und die Form der Gasaustrittsöffnung können selbstverständlich nicht nur, wie in den Ausführungsbeispielen gezeigt, in axialer Richtung ausgerichtet sein, sie können auch anders angeordnet sein, wie dies beispielsweise in der DE 198 20 240 A1 oder der DE 101 29 699 A1 dargestellt ist.

Die Begrenzung der Amplitude des durch das Plasma fließenden HF-Stroms dient nicht nur der kontrollierten Begrenzung der Penetrationstiefe der thermischen Effekte im Zielgewebe, sondern hat außerdem mehrere andere Vorteile, wie beispielsweise die Vermeidung zu hoher Temperaturen des Plasmas und hierdurch die Vermeidung der Karbonisation oder gar Pyrolyse des Zielgewebes, die Vermeidung thermischer Überlastungen des distalen Endes der Plasma-Sonde, insbesondere, wenn das Plasma direkt mit Kunststoff in Berührung kommt, wie beispielsweise bei Plasma-Sonden entsprechend DE 101 29 699, und außerdem die Möglichkeit, Störungen von Videosystemen und neuromuskulärer Reizungen zu vermeiden. Neuromuskuläre Reizungen werden bei Anwendung erfindungsgemäßer Plasma-Sonden durch den kapazitiven Widerstand vor der Ionisationselektrode vermieden, indem dieser Kapazitive Widerstand insbesondere niederfrequente Ströme verhindert.

Bezugszeichenliste

1    HF-Generator
2    Neutralelektrode
3    biologisches Gewebe
4    Zielgewebe
5    Endoskop
6    Arbeitskanal
7    Edelgasquelle
8    Innenwiderstand
9    Schlauch
10   Sonde
11   Zuleitung
12   Sondenleitung
13   Elektrode
14   Lichtbogen
15   Streukapazität
16   Streukapazität
20   Widerstandselement
21   Sondenleitungsdraht
22   Isolation
24   Elektrodenzuleitung
25   Verbindungsstelle

Patentansprüche

1.   Plasmaapplikator mit:

    einer Sonde (10);
    einer Anschlussleitung (11) und einer Sondenleitung (12);
    einer Elektrode (13);
    einem Widerstandselement (20) mit vorbestimmter Impedanz
    wobei die Sonde (10) rohr- oder schlauchförmig ausgebildet ist und zum Einführen in einen Arbeitskanal (6) eines flexiblen Endoskops (5) vorgesehen ist, wobei elektrische Energie von einem elektrochirurgischen HF-Generator (1) über die Anschluss- und Sondenleitung (11, 12) dem Widerstandselement (20) und der Elektrode (13) und weiter über einen Pfad oder Lichtbogen (14) aus ionisiertem Gas in ein biologisches Zielgewebe (4) und über eine neutrale Elektrode (2) zurück zum elektrochirurgischen HF-Generator (1) übertragen wird,

    **dadurch gekennzeichnet, dass**

    die Sondenleitung (12), das Widerstandselement (20) und die Elektrode (13) innerhalb der Sonde (10) angeordnet sind, wobei die Elektrode (13) am distalen Ende der Sonde (10) angeordnet ist, wobei das Widerstandselement (20) zwischen dem distalen Ende der Sondenleitung(12) und der Elektrode (13) angeordnet und derart dimensioniert ist, dass nach der Ionisierung des Gases durch Erzeugung eines Spannungsabfalls am Widerstandselement (20) eine Begrenzung eines Behandlungsstromes sichergestellt ist.

2.   Plasmaapplikator nach Anspruch 1,
     **dadurch gekennzeichnet, dass**

das Widerstandselement (20) eine Kapazität umfasst.

3. Plasmaapplikator nach einem der vorhergehenden Ansprüche,
**dadurch gekennzeichnet, dass**
das Widerstandselement (20) als Widerstand oder Kondensator ausgebildet ist.

4. Plasmaapplikator nach einem der vorhergehenden Ansprüche,
**dadurch gekennzeichnet, dass**
das Widerstandselement (20) einen Teilabschnitt der Sondenleitung (12) und/oder der Elektrode (13) umfasst.

5. Plasmaapplikator nach Anspruch 4,
**dadurch gekennzeichnet, dass**
das Widerstandselement (20) durch parallel geführte oder verdrillte oder koaxial angeordnete Teilabschnitte der Sondenleitung (12) und einer Zuleitung (24) zur Elektrode (13) und/oder der Elektrode (13) selbst gebildet ist.

6. Plasmaapplikator nach Anspruch 5,
**dadurch gekennzeichnet, dass**
die Teilabschnitte eine niedrige Induktivität aufweisen, wobei sie insbesondere als biphilare Anordnungen ausgebildet sind.

7. Plasmaapplikator nach einem der vorhergehenden Ansprüche,
**dadurch gekennzeichnet, dass**
das Widerstandselement eine Kapazität von 10 pF bis 1.000 pF umfasst.

8. Plasmaapplikator nach einem der vorhergehenden Ansprüche,
**dadurch gekennzeichnet, dass**
das Widerstandselement (20) ein Keramikmaterial als Isolierung und/oder Dielektrikum umfasst.

9. Plasmaappliktor nach einem der vorhergehenden Ansprüche,
**dadurch gekennzeichnet, dass**
die Elektrode (13) innerhalb der Sonde (10) derart angebracht ist, dass ein Edelgas, insbesondere Argon, als zu ionisierendes Gas in einen Raum zwischen der Elektrode (13) und dem Zielgewebe (4) zuführbar ist.

10. Plasmaapplikator nach Anspruch 8,
**dadurch gekennzeichnet, dass**
das Keramikmaterial pulverförmig ist.

**Claims**

1. A plasma applicator comprising:

   a probe (10);
   a connection line (11) and a probe line (12);
   an electrode (13);
   a resistive element (20) with predetermined impedance,
   wherein the probe (10) is tubular or hose-shaped and is provided for insertion into a working channel (6) of a flexible endoscope (5), wherein electrical energy is transferred from an electrosurgical HF generator (1) via the connection and probe line (11, 12) to the resistive element (20) and the electrode (13) and further via a path or an arc (14) of ionized gas into a biological target tissue (4) and via a neutral electrode (2) back to the electrosurgical HF generator (1),

   **characterized in that**

   the probe line (12), the resistive element (20), and the electrode (13) are arranged within the probe (10), wherein the electrode (13) is arranged at the distal end of the probe (10), wherein the resistive element (20) is arranged between the distal end of the probe line (12) and the electrode (13), and is configured such that a limit of a treatment current is ensured after gas ionization by generating a voltage drop.

2. The plasma applicator of claim 1,
   **characterized in that**
   the resistive element (20) comprises a capacitance.

3. The plasma applicator of one of the preceding claims,
   **characterized in that**
   the resistive element (20) is formed as resistor or capacitor.

4. The plasma applicator of one of the preceding claims,
   **characterized in that**
   the resistive element (20) comprises a segment of the probe line (12) and/or the electrode (13).

5. The plasma applicator of claim 4,
   **characterized in that**
   the resistive element (20) is formed by parallel-guided or twisted or coaxially-arranged segments of the probe line (12) and a supply line (24) to the electrode (13) and/or the electrode (13) itself.

6. The plasma applicator of claim 5,
   **characterized in that**
   the segments have a low inductance, wherein they are in particular formed as bifilar line arrangements.

7. The plasma applicator of one of the preceding claims,
**characterized in that**
the resistive element (20) has a capacitance of 10 pF to 1,000 pF.

8. The plasma applicator of one of the preceding claims,
**characterized in that**
the resistive element (20) comprises a ceramic material as insulation and/or a dielectric.

9. The plasma applicator of one of the preceding claims,
**characterized in that**
the electrode (13) is attached within the probe (10) such that an inert gas, in particular argon, is supplied as gas to be ionized into a chamber between the electrode (13) and the target tissue (4).

10. The plasma applicator of claim 8,
**characterized in that**
the ceramic material is a powder.

**Revendications**

1. Applicateur de plasma comprenant :

une sonde (10) ;
une ligne de raccordement (11) et une ligne de sonde (12) ;
une électrode (13) ;
un élément de résistance (20) présentant une impédance prédéfinie
la sonde (10) étant en forme de tuyau ou de tube et étant destinée à être introduite dans un canal de travail (6) d'un endoscope flexible (5), de l'énergie électrique étant transmise par un générateur électro-chirurgical HF (1) par l'intermédiaire des lignes de raccordement et de sonde (11, 12) à l'élément de résistance (20) et à l'électrode (13) et en plus, par l'intermédiaire d'un chemin ou d'un arc électrique (14) fait de gaz ionisé, dans un tissu biologique cible (4), et par l'intermédiaire d'une électrode neutre (2) à nouveau au générateur électro-chirurgical HF (1),

**caractérisé en ce que**

la ligne de sonde (12), l'élément de résistance (20) et l'électrode (13) sont disposés à l'intérieur de la sonde (10), l'électrode (13) étant disposée à l'extrémité distale de la sonde (10), l'élément de résistance (20) étant disposé entre l'extrémité distale de la ligne de sonde (12) et l'électrode (13) et étant dimensionné de telle manière qu'après l'ionisation du gaz, à la suite de la génération d'une chute de tension sur l'élément de résistance (20), une limitation d'un courant de traitement est garantie.

2. Applicateur de plasma selon la revendication 1,
**caractérisé en ce que**
l'élément de résistance (20) comprend une capacité.

3. Applicateur de plasma selon l'une quelconque des revendications précédentes,
**caractérisé en ce que**
l'élément de résistance (20) est réalisé sous la forme d'une résistance ou d'un condensateur.

4. Applicateur de plasma selon l'une quelconque des revendications précédentes,
**caractérisé en ce que**
l'élément de résistance (20) comprend une section de la ligne de sonde (12) et/ou de l'électrode (13).

5. Applicateur de plasma selon la revendication 4,
**caractérisé en ce que**
l'élément de résistance (20) est formé par des sections guidées parallèlement ou torsadées ou disposées coaxialement de la ligne de sonde (12) et d'une ligne d'amenée (24) à l'électrode (13) et/ou de l'électrode (13) elle-même.

6. Applicateur de plasma selon la revendication 5,
**caractérisé en ce que**
les sections présentent une faible inductance, lesdites sections étant réalisées en particulier sous la forme d'ensembles bifilaires.

7. Applicateur de plasma selon l'une quelconque des revendications précédentes,
**caractérisé en ce que**
l'élément de résistance comprend une capacité de 10 pF à 1000 pF.

8. Applicateur de plasma selon l'une quelconque des revendications précédentes,
**caractérisé en ce que**
l'élément de résistance (20) comprend un matériau céramique faisant office d'isolation et/ou de diélectrique.

9. Applicateur de plasma selon l'une quelconque des revendications précédentes,
**caractérisé en ce que**
l'électrode (13) à l'intérieur de la sonde (10) est montée de telle manière qu'un gaz rare, en particulier de l'argon, peut être amené en tant que gaz ionisant dans un espace situé entre l'électrode (13) et le tissu cible (4).

**10.** Applicateur de plasma selon la revendication 8,
**caractérisé en ce que**
le matériau céramique est pulvérulent.

Fig. 1

Fig. 2

Fig. 3

Fig. 4

Fig. 5

Fig. 6
STAND DER TECHNIK

Fig. 7
STAND DER TECHNIK

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4060088 A
- US 4781175 A
- DE 19839826
- EP 1148770 A2
- US 6958063 B1
- DE 19820240
- DE 10129699
- EP 1397082 A
- EP 0954246 A1
- DE 19820240 A1
- DE 10129699 A1

**In der Beschreibung aufgeführte Nicht-Patentliteratur**

- **G. FARIN ; K.E. GRUND.** Technology of Argon Plasma Coagulation with Particular Regard to Endoscopic Applications. *Endoscopic Surgery and Allied Technologies,* 1994, vol. 2 (1), 71-77
- **K.E. GRUND ; D. STOREK ; G. FARIN.** Endoscopic Argon Plasma Coagulation (APC): First Clinical Experiences in Flexible Endoscopy. *Endoscopic Surgery and Allied Technologies,* 1994, vol. 2 (1), 42-46
- **K.E. GRUND ; C. ZINDEL ; G. FARIN.** Argonplasmakoagulation in der flexiblen Endoskopie: Bewertung eines neuen therapeutischen Verfahrens nach 1606 Anwendungen. *Deutsche Medizinische Wochenschrift,* 1977, vol. 122, 432-438
- Principles of Electrosurgery, Laser, and Argon Plasma Coagulation with Particular Regard to Colonoscopy. **G. FARIN ; K.E. GRUND.** Colonoscopy; Principles and Practice. Blackwell Publishing, 2003, 393-409
- Technology of Argon Plasma Coagulation with Particular Regard to Endoscopic Applications. **VON G. FARIN ; K.E. GRUND.** Endoscopic Surgery and Allied Technologies. Thieme Verlag, 1994, vol. 2, 71-77